# EUROPEAN PATENT SPECIFICATION

(11) **EP 2 513 295 B1**
(45) Date of publication and mention of the grant of the patent: **10.08.2016**
(21) Application number: 10810877.0
(22) Date of filing: 16.12.2010
(51) Int. Cl.: C12N 5/00, C12N 5/071, G01N 33/53, G01N 33/50, C12Q 1/02

(54) **METHOD FOR THE THREE-DIMENSIONAL CO- CULTURE OF PODOCYTES AND ENDOTHELIAL CELLS AND RELATIVE IN VITRO CO- CULTURE SYSTEM**
VERFAHREN FÜR DIE DREIDIMENSIONALE KOKULTUR VON PODOZYTEN UND ENDOTHELZELLEN SOWIE ZUGEHÖRIGES IN-VITRO-KOKULTURSYSTEM
PROCÉDÉ DE COCULTURE TRIDIMENSIONNELLE DE PODOCYTES ET DE CELLULES ENDOTHÉLIALES ET SYSTÈME DE COCULTURE IN VITRO CORRESPONDANT

(30) Priority: 17.12.2009 IT MI20092216
(43) Date of publication of application: 24.10.2012
(73) Proprietor: Fondazione IRCCS Ca' Granda - Ospedale Maggiore Policlinico, 20122 Milano (IT)
(72) Inventor: RASTALDI, Maria Pia, I-20017 RHO (MI) (IT); LI, Min, I-20157 Milano (IT)
(74) Representative: Calogero, Ida
(86) International application number: PCT/IB2010/003298
(87) International publication number: WO 2011/073793

(56) References cited:
- EP-A1- 1 437 147
- US-A- 6 043 027
- WAYBILL P N ET AL: "Smooth Muscle Cell Proliferation in Response to Co-culture with Venous and Arterial Endothelial Cells", JOURNAL OF VASCULAR AND INTERVENTIONAL RADIOLOGY, VA LNKD- DOI:10.1016/S1051-0443(97)70575-X, vol. 8, no. 3, 1 May 1997 (1997-05-01), pages 375-381, XP026146817, ISSN: 1051-0443 [retrieved on 1997-05-01]
- MEGHNA WAGHRAY ET AL: "Hydrogen peroxide is a diffusible paracrine signal for the induction of epithelial cell death by activated myofibroblasts", THE FASEB JOURNAL : OFFICIAL PUBLICATION OF THE FEDERATION OF AMERICAN SOCIETIES FOR EXPERIMENTAL BIOLOGY MAY 2005 LNKD- PUBMED:15857893, vol. 19, no. 7, May 2005 (2005-05), pages 854-856, XP002583578, ISSN: 1530-6860
- PI-CHAO WANG ET AL: "Co-culture of glomerular epithelial cells and mesangial cells on collagen-gauze-fiber gel", BIOCHEMICAL ENGINEERING JOURNAL 20040815 NL LNKD- DOI:10.1016/J.BEJ.2003.11.002, vol. 20, no. 2-3, 15 August 2004 (2004-08-15), pages 149-154, XP002583579, ISSN: 1369-703X
- TAKUYA KANAZAWA ET AL: "A co-culture system for studies of paracrine effects of stromal cells on the growth of epithelial cells", METHODS IN CELL SCIENCE, KLUWER ACADEMIC PUBLISHERS, DO, vol. 14, no. 2, 1 January 1992 (1992-01-01), pages 59-62, XP009133739, ISSN: 1573-0603
- RAIMUND HIRSCHBERG ET AL: "Functional symbiosis between endothelium and epithelial cells in glomeruli", CELL AND TISSUE RESEARCH, SPRINGER, BERLIN, DE, vol. 331, no. 2, 13 November 2007 (2007-11-13), pages 485-493, XP019590706, ISSN: 1432-0878
- S J SHANKLAND ET AL: "Podocytes in culture: past, present, and future", KIDNEY INTERNATIONAL LNKD- PUBMED:17457377, vol. 72, no. 1, July 2007 (2007-07), pages 26-36, XP002583641, ISSN: 0085-2538

## Description

The present invention relates to a new three-dimensional co-culture method of podocytes and endothelial cells, and a relative in vitro co-culture system. Furthermore, the invention relates to the use of said co-culture system as a screening model of drugs or in vitro study of pathologies which affect the kidneys, and in particular the renal glomerular filtration barrier.

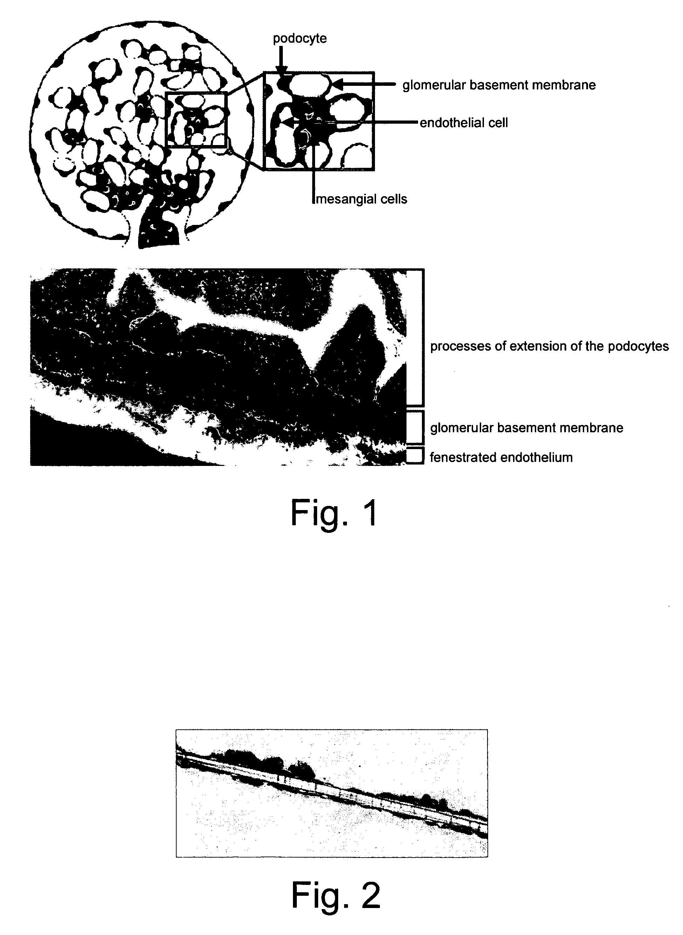

Conventional cell culture methods are generally used for defining the base properties of single cell types. Cells, however, live in a three-dimensional microenvironment which deeply influences their function. In particular, in the renal glomerulus, the podocytes adhere to the outer side of the glomerular basement membrane, whereas the endothelial cells adhere to its internal side. The three-dimensional organization of podocytes, basement membrane and endothelial cells allow the system to operate the filtration barrier function, as illustrated in Figure 1.

When the filtration barrier is damaged, proteins are lost in the urine. This phenomenon arises in primary diseases of the various glomerular components, and also as a consequence of extremely widespread systemic pathologies, such as obesity, diabetes and hypertension. In spite of the numerous efforts made in scientific research, and recent investigative progress, there are few explanations on the aetiology and pathogenesis of proteinuric pathologies with negative consequences on diagnostic and therapeutic possibilities, which have remained limited.

The possibility of recreating, in vitro, a structure similar to the filtration barrier would therefore be extremely useful in studying proteinuric pathologies and understanding the mechanisms which are at the basis of the protein loss on the part of the glomerular filter.

Numerous technical problems, however, have always prevented the creation of this system, above all due to the extreme differentiation and specialization of the cells composing it, and probably the interdependence of the various components of the system itself.

The cells which compose the filtration barrier are, in fact, extremely differentiated and specialized. It is also evident that a study of their function cannot disregard the context of the barrier itself due to various functional aspects.

Various authors in the past have used organotypic cultures of renal tissue in continuous perfusion [1-3], but the request for a simple co-culture system which reproduces the structure of the renal filtration barrier remains current.

Goligorsky et al. have described a sandwich co-culture method [4,5]. The podocyte line used consists of primary podocytes of rats transfected with SV-40. The endothelial line used is HUVEC obtained by Clonetics Corp. The podocytes are grown on the bottom of 24-well plates, and are subsequently covered by Matrigel (extracellular matrix, Fisher Scientific) which is gelified at 37°C for 30 minutes. After adding the medium, the endothelial cells are added to each well, completing the sandwich. With respect to the physiological configuration of the filtration barrier, it is evident that the system differs in two substantial elements: a) the podocyte cell is compelled to adhere to both the base of the well and to the matrix covering it; b) there is no possibility of reproducing glomerular filtration, as there is no supernatant on the podocyte side. In the recent work published by Hirschberg R. et al. [6], the co-culture system is thus composed: the podocytes (conditional immortalized mouse line obtained from the so-called Immortomouse) are cultivated on membranes positioned in 96-well plates and induced to differentiation. The endothelial cells (in this case they are differentiated endothelial cells starting from circulating progenitors) are cultivated on the base of wells in 96-well plates. The membranes with the podocytes are then positioned in the wells containing the endothelial cells. It is not clear whether the membrane rests on the endothelial cells or whether it remains suspended, but in both cases the system is clearly different from the physiological condition of the glomerular filter, as the space ratios between the elements forming it are altered.

Another co-culture system is described in EP1437147A1, which is mainly designed for the growth of alveolar epithelial cells and endothelial cells. The experimental reproduction of this system, however, effected by the authors of the present invention according to the method described in EP1437147A1, did not allow an artificial glomerulus to be reconstructed, i.e. the co-culture system when podocytes and glomerular endothelial cells are used, in view of the fact that no VEGF pre-treatment of endothelial cells is contemplated.

Waybill P. N. et al., 1997 disclose a co-culture system comprising a polycarbonate membrane for culturing endothelial and smooth muscle cells on alternate sides of the membrane. Again, the pre-treatment of the endothelial cells with VEGF is not mentioned at all.

On the basis of what is indicated above, there is an evident necessity for availing of a new co-culture method of podocytes and endothelial cells, and a relative three-dimensional in vitro system which reproduces in a simplified but complete manner, the characteristics and space ratios of the glomerular filter, and allows the application of numerous research methods to both of the populations in the culture and their respective supernatants, overcoming the disadvantages of the techniques so far used.

The authors of the present invention have set up a new three-dimensional co-culture method of podocytes and endothelial cells, together with the relative in vitro co-culture system.

An object of the present invention therefore relates to an in vitro co-culture system of podocytes and endothelial cells comprising a solid support containing a first culture medium inside which a semipermeable membrane consisting of plastic material with pores having a diameter ranging from 0.3 µM to 1.2 µM, preferably 1 µM, is kept in suspension through a second solid support, said membrane being coated on both sides with collagen type IV, wherein on the lower side of the membrane a microvascular endothelial mammal cell line is grown in the first culture medium and on the upper side of the membrane a mammal podocyte line is placed in culture in a second culture medium, wherein said microvascular endothelial mammal cell line is obtained by growing it in the presence of VEGF at a concentration of 3 to 50 ng/ml for one week before plating podocytes.

The membrane is preferably polyethyleneterephthalate (PET). In a particularly preferred embodiment of the invention, the Millipore membrane (Millicell Hanging Cell Culture Insert) having pores with a diameter of 1 µM, is used.

The line of mammal podocytes is preferably the immortalized line obtained from the authors of the present invention, from the mouse Immortomouse (Charles River, St Louis, MO, USA), a transgenic mouse for a sensitive temperature variant of "SV40 large T antigen" under the control of the promoter H-2Kb interferon-gamma-inducible (as described in [7]).

In alternative embodiments, the podocyte line can be mutated (i.e. by means of gene silencing), or primary podocyte cultures can be used coming from genetically normal animals or transgenic animals.

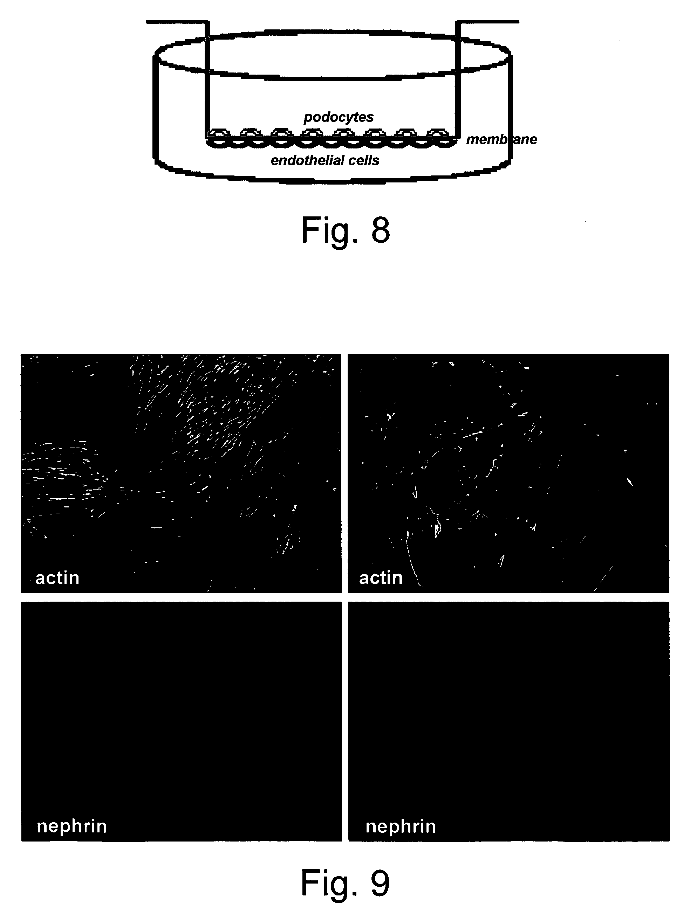

In a preferred embodiment of the invention, the solid support is a multi-well plate. Figure 8 schematically illustrates the configuration of the co-culture system of podocytes and endothelial cells inside a well.

According to an alternative embodiment, the podocyte line can be replaced with a line of pericytes to simulate, in vitro, the system of pericytes microvascular endothelial cells which exists on the level of all the capillaries of the organism.

According to another alternative embodiment, the system according to the invention also comprises a third cell population situated on the bottom of the First solid support, and selected according to the tissue to be reproduced from mesangial cells (in the case of renal glomerulus), fibroblasts (connective tissue), astrocytes (hemato-encephalic barrier), or specific parenchymal cells (myocardiocytes, hepatocytes, neuronal cells, etc.).
A further object of the present invention relates to a method for the in vitro co-culture of podocytes and endothelial cells comprising the following phases: a) culture of a microvascular endothelial mammal cells line on the lower side of a semi-permeable membrane coated on both sides with collagen type IV consisting of plastic material with pores of diameter between 0.3 µM and 1.2 µM kept in suspension inside a solid support containing a first culture medium in the presence of VEGF at a concentration of between 3 and 50 ng/ml, preferably 5 ng/ml, for one week;
b) culture of a mammal podocytes line under "permissive conditions" in the presence of gamma-interferon at a temperature of 33 °C (7), for one week;
c) transfer of the mammal podocytes line obtained from step b) on the upper side of the membrane of step a) and culture in a second culture medium under "non-permissive conditions", in the absence of gamma-interferon at a temperature of 37°C (7);
d) co-culture of the two cell lines in the two environments separated by the membrane.

Said solid support is preferably a multi-well plate.

According to a preferred embodiment of the method of the invention, said membrane is PET with pores having a diameter of 1 µM. In a particularly preferred embodiment of the invention, the membrane Millipore (Millicell Hanging Cell Culture Insert) having pores with a diameter equal to 1 µm, is used.

The mammal podocytes line is preferably that obtained in our laboratory from the mouse Immortomouse (Charles River, St Louis, MO, USA), a transgenic mouse for a sensitive temperature variant of "SV40 large T antigen" under the control of the promoter H-2Kb interferon-gamma-inducible [7]. In alternative applications, the podocyte line can be mutated (i.e. by means of gene silencing), or primary podocyte cultures can be used coming from genetically normal animals or transgenic animals.

When an interferon-gamma-inducible conditionally immortalized podocytes line previously grown for a week in "permissive conditions", in the presence of interferon-gamma at 33°C, is used, the culture in a second medium of step c) in "non-permissive conditions" takes place in the absence of interferon-gamma and at 37°C.

When a primary culture is used, the initial phase comprises the isolation of the glomeruli, which are put in culture until the podocytes begin to grow. Cells and glomeruli are then trypsinized and the glomeruli are eliminated by filtration, to obtain the primary podocyte culture (method described in [7]).

According to a preferred embodiment of the invention, the line of microvascular endothelial mammal cells is the murine line ATCC Number CRL-2586™.

The method according to the invention can also comprise the introduction of a third cell line on the bottom of the solid support, selected according to the tissue which is to be reproduced from mesangial cells (in the case of renal glomerulus), fibroblasts (connective tissue), astrocytes (hemato-encephalic barrier), or specific parenchymal cells (myocardiocytes, hepatocytes, neuronal cells, etc.).

According to an alternative embodiment of the method of the invention, the line of podocytes can be substituted with a line of pericytes and the culture medium of step a) contains PDGF-B (specific growth factor for pericytes) instead of VEGF.

The invention also relates to the use of the in vitro co-culture system as defined above in a screening method of drugs or substances suitable for modifying the functionality of the renal glomerulus comprising the evaluation of one or more parameters selected from permeability of the capillary barrier, cell morphology, immunodetection of the specific cell expression markers and markers released in the supernatants of the cell populations.

It is evident that the advantage of operating in a three-dimensional context, even if simplified with respect to the in vivo structure, is essentially due to the possibility of applying stimuli and variations on one side of the membrane and studying the consequences produced on the other side.

The evaluation of the permeability barrier can be effected through spectrophotometric measurements of the transmembrane passage of proteins or polymers with a defined molecular weight (such as albumin or dextrans), also conjugated with a fluorescence marker.

The morphology of the cells of the co-culture system according to the invention or their confluence degree can be easily controlled by means of optical microscopy, transmission and scanning electron microscopy.

The system also enables the qualitative evaluation of specific markers of the two cell populations by means of immunocytochemistry, immunofluorescence, and immunogold electron microscopy. In order to quantitatively evaluate the above expression markers it is also possible to carry out quantitative methods, such as the so-called "in cell ELISA" which allows information to be obtained on the protein expression relating to the number of cells present on the membrane.

Each type of biochemical or molecular test (e.g. ELISA e Western Blot), can be subsequently applied to the study of the respective supernatants.

Finally, the invention relates to the use of the co-culture system according to the invention, as an in vitro study model of the dysfunctions or pathologies affecting the renal glomeruli.

The three-dimensional co-culture system of podocytes and endothelial cells according to the invention can therefore be effectively applied in pharmacological studies, in the discovery of diagnostic markers, and in analyses of biological samples coming from individual subjects, with the perspective of a personalization of the diagnosis and treatment.

The present invention will now be described for illustrative but non-limiting purposes, according to its preferred embodiments with particular reference to the figures of the enclosed drawings, wherein:
figure 1 shows the structure of the renal glomerulus, an entanglement of glomerular capillaries whose basal membrane is externally covered by the extensions of the podocytes (foot processes) and internally by the endothelial cells, forming the filtration barrier. The glomerular capillaries are sustained by the mesangial axis, composed of mesangial cells and extracellular matrix.
figure 2 shows the image obtained by optical microscopy of the co-culture system, whereby it is possible to control the presence of the two cell populations on the two sides of the membrane. A toluidine blue-coloured semifine section is represented in the figure.
figure 3 shows the image of the co-culture system obtained by transmission electron microscopy. Transmission electron microscopy allows the morphological details of the two cell types to be examined. In the image the interaction between cell extensions of podocytes is evident.
figure 4 shows the image of the co-culture system obtained by scanning electron microscopy. With scanning electron microscopy, it is possible to add further information on the morphology and cell density. An image obtained from the side of the endothelial cells is represented in the figure.
figure 5 shows the expression of CD31 on the part of the endothelial cells grown in co-culture (IF, 200X) by means of immunofluorescence.
figure 6 shows the expression of VCAM-1 on the part of the endothelial cells, put in co-culture with primary podocytes coming from the null mouse for Rab3A (IHC, 400X) by immunohistochemistry.
figure 7 shows the Western Blots carried out on podocyte line cultures after two weeks of growth under non-permissive conditions. The differentiated cells are positive for specific podocyte markers of mature cells.
figure 8 schematically shows the co-culture system of podocytes and endothelial cells according to the invention.
figure 9 shows the differences between cells grown on collagen type IV and collagen type I. The image shows the phenotype change of the podocyte line depending on the type of collagen on which the cells are grown. The images on the left are examples of cells grown on type IV collagen: the actin is organized in stress fibres which pass through the cell body and continue along the extensions. Nefrin is prevalently expressed along the cell extensions. The two images on the right show the podocytes grown on type I collagen. The reduction in stress fibres and general remodelling of actin can be observed. Furthermore, nefrin is exclusively present in the cell body, whereas it is absent in the cell extensions.

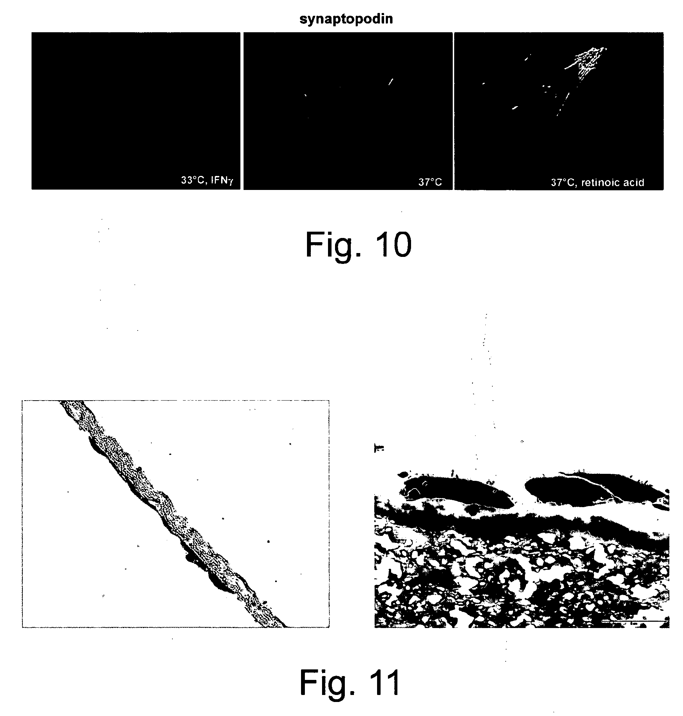

figure 10 shows the expression of specific podocyte proteins in the maturation phases of the conditional immortalized podocyte mouse line used, and the further action favouring cell differentiation on the part of retinoic acid. Synaptopodin, a podocyte marker present in mature podocytes, is completely absent in cells cultivated under "permissive conditions" (image on the left). The positivity for synaptopodin is evident in cells matured means of by temperature variation and absence of interferon-gamma (image in the centre), and the marker is more fully expressed if the cells are differentiated in the presence of retinoic acid (image on the right).
figure 11 illustrates the use of a different membrane (PTFE membrane, polytetrafluoroethylene) from that selected and used according to the present invention.

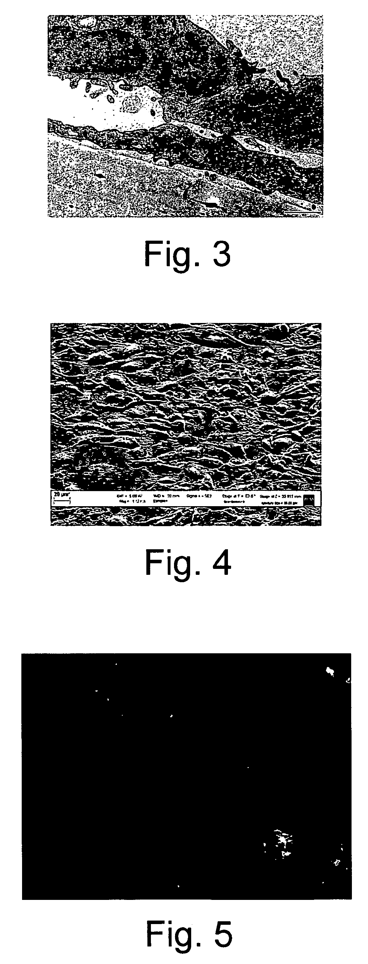

It should be noted how the structure of the membrane is less homogeneous with respect to that represented in figures 2 and 3, and prevents the adhesion of the podocyte population. The characteristics of non-homogeneity of the membrane and poor resistance to processing methods are particularly evident in the image in transmission electron microscopy.

An embodiment example of the co-culture system, object of the present invention, is provided hereunder for illustrative but non-limiting purposes of the present invention.

### EXAMPLE 1

### MATERIALS

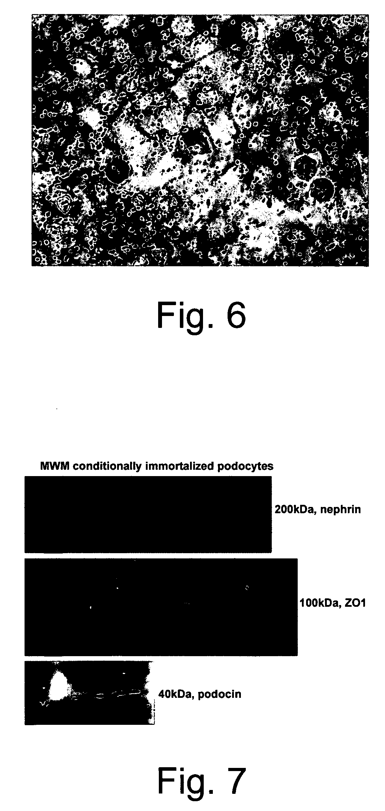

The following were used for obtaining the co-culture system:
a) A conditional immortalized mouse podocyte line obtained in our laboratory [method described in 7], following, with some modifications, the method described by Mundel P et al [8]. In short, the glomeruli were isolated from the kidneys of transgenic mice H-*2K^{b}*-tsA58 (Immortomouse, Charles River, St Louis, MO, USA) aged 6-8 weeks. These are transgenic mice for a sensitive temperature variant of "SV40 large T antigen" under the control of the promoter H-2Kb interferon-gamma-inducible. The glomeruli were separated from the renal tissue by sieving the tissue through filters with a decreasing pore diameter (from 100 to 36µm) and grown in a standard medium at 37°C. When the podocyte cells began to grow, cells and glomeruli were detached by treatment with trypsin and the glomeruli eliminated by filtration. The cells were re-plated and propagated at 33°C in a medium containing 20U/ml of recombinant mouse interferon-gamma (so-called "permissive conditions"). The cells were then purified according to the limiting dilution method [8], i.e. cultivated at decreasing concentrations (10, 1, 0.1 cells/vial). The clones obtained were characterized by immunocytochemistry and western blot and only the 5 clones which expressed WT1 and nefrin in over 90% of the cells, were selected and propagated. A cell aliquot from each clone was brought to complete maturation, whereas most of the cells were frozen. The maturation was obtained by growth under "non-permissive conditions", i.e. at 37°C in a medium not containing interferon-gamma (7).
   This podocyte line is therefore characterized by a high proliferative capacity when brought to 33°C in the presence of interferon-gamma (so-called "permissive conditions") (7). The cells, brought to 37°C in the absence of interferon for about two weeks (so-called "non-permissive conditions") differ in mature podocytes, having cell extensions and expressing the complete panel of specific markers of these cells, such as nefrin, podocin, synaptopodin, alpha-actinin (Figure 7).
   Alternatively, the podocyte line can be substituted by a culture of primary podocytes. The latter offer the advantage of a better differentiation which makes them functionally more similar to the in vivo situation, and allow cells coming from transgenic animals of interest to be used. The method used (see reference [7]) envisages the isolation of glomeruli from newly-born animals (maximum 10 days of age), and was borrowed from methods for obtaining cultures of primary neurons. Potential disadvantages, which should be taken into consideration, are determined by the shorter cell survival and necessity for a complete characterization of the cells at each isolation.
b) a mouse endothelial cell line (EOMA-Mus musculus, ATCC-CRL-2586), characterized by functional aspects typical of microvascular endothelial cells. This is a particularly important detail, as microvascular endothelial cells differ in numerous functional aspects from cells lines commonly used, such as HUVEC, which derive from the venous endothelium, or from other derivation lines from vessels having a greater calibre than capillaries. It is difficult to replace the cell line with primary glomerular cells, as the glomerular endothelium is particularly differentiated and non-proliferous when put in a culture.
c) a semi-permeable membrane with pores having a diameter ranging from 0.3 µm to 1.2 µm, preferably a membrane of PET with pores of 1 µm (Millicell Hanging Cell Culture Insert, Millipore) which can be allocated in suspension inside wells in the culture plates, so that the base of the membrane is suspended in the medium and does not touch the bottom of the well. The selection of the membrane was determined by particular properties of the material forming it (PET) which is ideal for the growth of the cell types considered herein.
   PET was selected as it is the material used in vascular grafts (12). The use of a polycarbonate membrane was discarded as this material is not transparent to microscopes (Millicell Technical Guide, 2004, Lit. No.: TN2004EN00, page 4 Millipore, http://www.millipore.com/publications.nsf/a736,64f9f981a f8c852569b9005b4eee/2984f0525bf6e23285256e820061bd4e/$F ILE/TN2004EN00.pdf). The use of a PTFE membrane is not suitable for the purposes of electron microscopy, as indicated in figure 11.
   Furthermore the same material offers various advantages such as an improved consistency and resistance when the system is removed from the well and processed by transmission electron microscopy, the improved shear quality of ultrathin sections (the membrane does not exfoliate), and the excellent visibility of the cells in both bright field microscopy and in immunofluorescence and immunocytochemistry.
   The use of a different type of membrane led to a series of drawbacks, as represented in Figure 11.
d) the membrane is covered on both sides with type IV collagen (12). The choice of collagen is extremely important, as type IV collagen is that which is physiologically present in basal membranes of glomerular capillaries. The use, for example, of type I collagen produces important variations in the cell phenotype, as represented in Figure 9.

### METHODS

When placed on the two sides of the membrane, the two cell types are cultivated in specific mediums which allow their differentiation and maturation. In particular, Vascular Endothelial Growth Factor (VEGF) [9] is added to the culture medium of the endothelial cells and the endothelial cells are left to grow alone on the lower side of the membrane for a week. Only at this point are the podocytes added under "non-permissive conditions".

### RESULTS

The system conceived by the present invention is schematized in Figure 8. The system consists of a membrane suspended in a well of a plate for cell cultures. The endothelial cells are grown on the lower side of the membrane, whereas the podocytes are put in culture on the upper side.

The endothelial cells are put in culture on the lower side of the membrane, and the growth factor VEGF is added to the culture medium at a concentration of 5ng/ml.

This phase is critical as VEGF is produced by the podocytes in the glomerulus, and the endothelial cells have the specific receptors of this growth factor. Knock-out animals for VEGF have endothelial alterations which do not allow a normal development of the glomerular structure [10] and it has been demonstrated that the addition of VEGF to endothelial cell lines is capable of causing the fenestration typical of the glomerular endothelium [11].

After a week, the podocytes (maintained up until this moment under "permissive conditions", i.e. at 33°C with the addition of interferon-gamma) are placed on the upper side of the membrane and are left to differentiate for two weeks under "non-permissive conditions" (i.e. in the absence of interferon and at a temperature of 37°C).

In a further variant, retinoic acid is added to the medium which covers the podocytes on the membrane, which allows a further differentiation of the podocyte cell, making it even more similar both morphologically and functionally to the primary cell and in vivo cell (Figure 10).

The system is formed so as to keep the two cell environments and respective supernatants separate. The communication between the two micro-environments takes place exclusively through the membrane itself.

The presence of the cells and their confluence degree can be easily controlled by means of optical microscopy (Figure 2), transmission electron microscopy (Figure 3) and scanning electron microscopy (Figure 4).

The system allows the study of the two cell components by means of immunofluorescence (Figure 5) and immunohistochemistry (Figure 6).

In short, the cells are fixed in cold acetone or paraformaldehyde, depending on the markers to be analyzed. This is followed by a permeabilization with Triton, a pre-incubation with BSA, the cells are then sequentially incubated with the primary antibody, the secondary marked antibody, and if necessary the detection system. At this point, the membrane is detached from the support and adhered to a plate, so that the cell type of interest is on the surface. The plate is covered with a cover glass plate and the results are analyzed by optical or fluorescence microscopy.

It is also possible to substitute the cell lines indicated herein with cells obtained from transgenic animals (Figure 6). There are currently numerous proteinuric animal models obtained by mutation of podocyte molecules. The co-culture system according to the invention can be used complementarily with respect to the in vivo study of animals, as it allows the co-culture of primary podocytes coming from the experimental model in a system which reproduces the situation of the filtration barrier in vitro.

Alternatively, with respect to the primary culture coming from mutated animals, the conditional immortalized cell line preferably used herein can be further mutated or silenced into genes of interest. The same also applies to the endothelial line. Furthermore, modified/mutated components of the glomerular basal membrane can substitute/be added to type IV collagen, to reproduce other pathological situations.

### BIBLIOGRAPHY

[1] Avner ED, Villee DB, Schneeberger EE, Grupe WE. An organ culture model for the study of metanephric development. J Urol 1983; 129: 660-664.
[2] Avner ED, Sweeney WE Jr. Polypeptide growth factors in metanephric growth and segmental nephrin differentiation. Pediatr Nephrol 1990; 4: 372-377.
[3] Kloth S, Aigner J, Kubitza M, Schmidbauer A, Gerdes J, Moll R, Minuth WW. Development of renal podocytes cultured under medium perifusion. Lab Invest 1995; 73: 294-301.
[4] Chen J, Braet F, Brodsky S, Weinstein T, Romanov V, Noiri E, Goligorsky MS. VEGF-induced mobilization of caveolae and increase in permeability of endothelial cells. Am J Physiol Cell Physiol. 2002; 282: C1053-63.
[5] Kim BS, Chen J, Weinstein T, Noiri E, Goligorsky MS. VEGF expression in hypoxia and hyperglycemia: reciprocal effect on branching angiogenesis in epithelial-endothelial co-cultures. J Am Soc Nephrol. 2002; 13: 2027-36.
[6] Hirschberg R, Wang S, Mitu GM. Functional symbiosis between endothelium and epithelial cells in glomeruli. Cell Tissue Res. 2008; 331: 485-93.
[7] Giardino L, Armelloni S, Corbelli A, Mattinzoli D, Zennaro C, Guerrot D, Tourrel F, Ikehata M, Li M, Berra S, Carraro M, Messa P, Rastaldi MP. Podocyte glutamatergic signaling contributes to the function of the glomerular filtration barrier. J. Am. Soc. Nephrol. 2009; 20:1929-40.
[8] Mundel P, Reiser J, Zúñiga Mejía Borja A, Pavenstädt H, Davidson GR, Kriz W, Zeller R: Rearrangements of the cytoskeleton and cell contacts induce process formation during differentiation of conditionally immortalized mouse podocyte cell lines. Exp Cell Res 236: 248-258, 1997.
[9] Satchell SC, Tasman CH, Singh A, Ni L, Geelen J, von Ruhland CJ, O'Hare MJ, Saleem MA, van den Heuvel LP, Mathieson PW. Conditionally immortalized human glomerular endothelial cells expressing fenestrations in response to VEGF. Kidney Int 2006: 69:1633-1640.
[10] Eremina V, Sood M, Haigh J, Nagy A, Lajoie G, Ferrara N, Gerber HP, Kikkawa Y, Miner JH, Quaggin SE. Glomerular-specific alterations of VEGF-A expression lead to distinct congenital and acquired renal diseases. J Clin Invest. 2003 Mar;111(5):707-16).
[11] Satchell SC, Tasman CH, Singh A, Ni L, Geelen J, von Ruhland CJ, O'Hare MJ, Saleem MA, van den Heuvel LP, Mathieson PW. Conditionally immortalized human glomerular endothelial cells expressing fenestrations in response to VEGF. Kidney Int. 2006: 69: 1633-1640).
[12] Liu Y, He T, Song H, Gao C. Layer-by-layer assembly of biomacromolecules on poly(ethylene terephthalate) films and fiber fabrics to promote endothelial cell growth. J Biomed Mater Res 2007; 81A: 692-704.

## Claims

1. An in vitro co-culture system of podocytes and endothelial cells comprising a solid support containing a first culture medium inside which a semi-permeable membrane of PET with pores of diameter between 0.3 µm and 1.2 µm is kept in suspension through a second solid support, said membrane being coated on both sides with collagen type IV, wherein on the lower side of the membrane a microvascular endothelial mammal cell line is grown in the first culture medium and on the upper side of the membrane a mammal podocyte line is placed in culture in a second culture medium; wherein said microvascular endothelial mammal cell line is obtained by growing it in the presence of VEGF at a concentration of 3 to 50 ng/ml for one week before plating podocytes.

2. The system according to claim 1, wherein said VEGF is added at a concentration of 5 ng/ml for one week before plating podocytes.

3. The system according to each of claims 1-2, wherein said membrane has pores having a diameter equal to 1 µm.

4. The system according to each of claims 1-3, wherein said line of mammal podocytes is conditionally immortalized.

5. The system according to each of claims 1-4, wherein said solid support is a multi-well plate.

6. The system according to each of claims 1-5, also comprising a third cell population on the bottom of the first solid support selected from the group consisting of mesangial cells, fibroblasts, astrocytes and parenchymal cells.

7. A method for the in vitro co-culture of podocytes and endothelial cells comprising the following steps:
a) culture of a microvascular endothelial mammal cells line on the lower side of a semi-permeable membrane of PET coated on both sides with collagen type IV with pores having a diameter ranging from 0.3 µm to 1.2 µm kept in suspension inside a solid support containing a first culture medium in the presence of VEGF at a concentration of 3 to 50 ng/ml, preferably 5 ng/ml, for one week before plating podocytes;
b) culture of a line of mammal podocytes in the presence of interferon-gamma at 33°C for one week;
c) transfer of the mammal podocytes line obtained from step b) on the upper side of the membrane of step a) and culture in a second medium in the absence of interferon-gamma and at a temperature of 37°C;
d) co-culture of the two cell lines in the two environments separated by the membrane.

8. The method according to claim 7, wherein said solid support is a multi-well plate.

9. The method according to each of claims 7-8, wherein said membrane is made of PET with pores having a diameter of 1 µm.

10. The method according to each of claims 7-9, wherein said line of podocytes is conditionally immortalized.

11. Method according to claim 10, wherein when an interferon-gamma-inducible conditionally immortalized podocytes line grown in the presence of interferon-gamma at 33°C, is used, the culture in a second medium of step c) takes place in the absence of gamma interferon and at 37°C.

12. The method according to each of claims 7-11, wherein under item b) a primary culture of mammal podocytes is used.

13. Method according to each of claims 7-12, wherein said microvascular endothelial cells line is the murine line ATCC CRL-2586.

14. The method according to each of claims 7-13, comprising the introduction of a third cell line on the bottom of the solid support selected from the group consisting of mesangial cells, fibroblasts, astrocytes and parenchymal cells.

15. Use of the in vitro co-culture system as defined according to claims 1-6, in a screening method of drugs or substances suitable for modifying the functionality of the renal glomerulus comprising the evaluation of one or more parameters selected from cell permeability, cell morphology, immunodetection of the expression markers released in the supernatants of the cell populations.

16. Use of the in vitro co-culture system according to claim 15, wherein said cell permeability is evaluated through the use of albumin or dextrans, also marked.

17. Use of the in vitro co-culture system according to claim 15, wherein the cell morphology is analysed through optical or electronic microscopy.

18. Use of the in vitro co-culture system according to claim 15, wherein the expression markers released in the supernatants are detected through the ELISA or Western blot test.

19. Use of the in vitro co-culture system as defined according to claims 1-6, as an in vitro study model of the dysfunctions or pathologies affecting the renal glomeruli.

20. Use of the in vitro co-culture system as defined according to claims 1-6, in a screening method of drugs or substances suitable for modifying the functionality of the renal glomerulus comprising the evaluation of one or more parameters selected from cell permeability, cell morphology, immunodetection of the expression markers released in the supernatants of the cell populations.

## Patentansprüche

1. In-vitro-Kokultursystem von Podozyten und Endothelzellen, umfassend einen festen Träger, der ein erstes Kulturmedium enthält, in dem eine halbdurchlässige Membran bestehend aus PET mit Poren eines Durchmessers zwischen 0,3 µm und 1,2 µm in Suspension gehalten wird durch einen zweiten festen Träger, wobei die Membran beidseitig mit Kollagen des Typs IV beschichtet ist, wobei auf der unteren Seite der Membran eine mikrovaskuläre Säugetier-Endothelzelllinie im ersten Kulturmedium gezüchtet wird und auf der oberen Seite der Membran eine Säugetier-Podozytenzelllinie in einem zweiten Kulturmedium in Kultur gegeben wird; wobei die mikrovaskuläre Säugetier-Endothelzelllinie durch Züchten in Gegenwart von VEGF in einer Konzentration von 3 bis 50 ng/ml über einen Zeitraum von einer Woche vor dem Ausplattieren von Podozyten gewonnen wird.

2. System nach Anspruch 1, wobei das VEGF in einer Konzentration von 5 ng/ml über einen Zeitraum von einer Woche vor dem Ausplattieren von Podozyten hinzugegeben wird.

3. System nach jedem der Ansprüche 1 bis 2, wobei die Membran Poren eines Durchmessers von 1 µm aufweist.

4. System nach jedem der Ansprüche 1 bis 3, wobei die Säugetier-Podozytenzelllinie bedingt immortalisiert ist.

5. System nach jedem der Ansprüche 1 bis 4, wobei der feste Träger eine Multi-Well-Platte ist.

6. System nach jedem der Ansprüche 1 bis 5, ferner umfassend eine dritte Zellpopulation auf dem Boden des ersten festen Trägers, ausgewählt aus der Gruppe bestehend aus mesangialen Zellen, Fibroblasten, Astrozyten und parenchymalen Zellen.

7. Verfahren für die In-vitro-Kokultur von Podozyten und Endothelzellen umfassend folgende Schritte:
a) Kultivieren einer mikrovaskulären Säugetier-Endothelzelllinie auf der unteren Seite einer halbdurchlässigen Membran aus PET, die beidseitig mit Kollagen des Typs IV mit Poren eines Durchmessers zwischen 0,3 µm und 1,2 µm beschichtet ist, die in einem festen Träger, enthaltend ein erstes Kulturmedium in Anwesenheit von VEGF in einer Konzentration von 3 bis 50 ng/ml, vorzugsweise 5 ng/ml, über einen Zeitraum von einer Woche vor dem Ausplattieren von Podozyten in Suspension gehalten wird;
b) Kultivieren einer Säugetier-Podozytenzelllinie in Anwesenheit von Interferon-gamma bei 33 °C über einen Zeitraum von einer Woche;
c) Übertragen der in Schritt b) auf der oberen Seite der Membran aus Schritt a) erhaltenen Säugetier-Podozytenzelllinie und Kultivieren in einem zweiten Medium in Abwesenheit von Interferon-gamma und bei einer Temperatur von 37 °C;
d) Kokultivieren der beiden Zelllinien in den beiden durch die Membran getrennten Umgebungen.

8. Verfahren nach Anspruch 7, wobei der feste Träger eine Multi-Well-Platte ist.

9. Verfahren nach jedem der Ansprüche 7 bis 8, wobei die Membran aus PET hergestellt ist mit Poren eines Durchmessers von 1 µm.

10. Verfahren nach jedem der Ansprüche 7 bis 9, wobei die Podozytenzelllinie bedingt immortalisiert ist.

11. Verfahren nach Anspruch 10, wobei wenn eine Interferon-gamma-induzierbare, bedingt immortalisierte, in Anwesenheit von Interferon-gamma bei 33 °C gezüchtete Podozytenzelllinie verwendet wird, findet das Kultivieren in einem zweiten Medium aus Schritt c) in Abwesenheit von Gamma-Interferon und bei 37 °C statt.

12. Verfahren nach jedem der Ansprüche 7 bis 11, wobei unter Punkt b) eine Primärkultur von Säugetier-Podozyten verwendet wird.

13. Verfahren nach jedem der Ansprüche 7 bis 12, wobei die mikrovaskuläre Endothelzelllinie die Mäusezelllinie ATCC CRL-2586 ist.

14. Verfahren nach jedem der Ansprüche 7 bis 13, umfassend die Einführung einer dritten Zelllinie auf dem Boden des festen Trägers, ausgewählt aus der Gruppe bestehend aus mesangialen Zellen, Fibroblasten, Astrozyten und parenchymalen Zellen.

15. Verwendung des In-vitro-Kokultursystems nach den Ansprüchen 1 bis 6 in einem Screening-Verfahren für Arzneistoffe oder Substanzen, die geeignet sind zur Veränderung der Funktionsweise des Nierenglomerulus, umfassend die Evaluierung eines oder mehrerer Parameter, ausgewählt aus Zellpermeabilität, Zellmorphologie, Immunnachweis der Expressionsmarker, die in den überständigen Zellpopulationen abgegeben werden.

16. Verwendung des In-vitro-Kokultursystems nach Anspruch 15, wobei die Zellpermeabilität mittels Verwendung von Albumin oder ebenfalls markierten Dextranen evaluiert wird.

17. Verwendung des In-vitro-Kokultursystems nach Anspruch 15, wobei die Zellmorphologie mittels optischer oder elektronischer Mikroskopie analysiert wird.

18. Verwendung des In-vitro-Kokultursystems nach Anspruch 15, wobei die in den Überständen abgegebenen Expressionsmarker mittels ELISA-Test oder Western-Blot-Test nachgewiesen werden.

19. Verwendung des In-vitro-Kokultursystems nach den Ansprüchen 1 bis 6 als In-vitro-Studienmodell der Dysfunktionen oder Pathologien, die die Nierenglomeruli betreffen.

20. Verwendung des In-vitro-Kokultursystems nach den Ansprüchen 1 bis 6, in einem Screening-Verfahren für Arzneistoffe oder Substanzen, die geeignet sind zur Veränderung der Funktionsweise des Nierenglomerulus, umfassend die Evaluierung eines oder mehrerer Parameter, ausgewählt aus Zellpermeabilität, Zellmorphologie, Immunnachweis der Expressionsmarker, die in den überständigen Zellpopulationen abgegeben werden.

## Revendications

1. Système de coculture in vitro de podocytes et de cellules endothéliales comprenant un support solide contenant un premier milieu de culture à l'intérieur duquel une membrane semi-perméable constituée de PET avec des pores de diamètre entre 0,3 µm et 1,2 µm est maintenue en suspension par le biais d'un deuxième support solide, ladite membrane étant enduite des deux côtés de collagène de type IV, dans lequel, sur le côté inférieur de la membrane, une lignée de cellules de mammifère endothéliales microvasculaires est développée dans le premier milieu de culture et, sur le côté supérieur de la membrane, une lignée de podocytes de mammifère est placée en culture dans un deuxième milieu de culture ; dans lequel ladite lignée de cellules de mammifère endothéliales microvasculaires est obtenue en la développant en présence de VEGF à une concentration de 3 à 50 ng/ml pendant une semaine avant la préparation de podocytes en plaques.

2. Système selon la revendication 1, dans lequel ledit VEGF est ajouté à une concentration de 5 ng/ml pendant une semaine avant la préparation de podocytes en plaques.

3. Système selon chacune des revendications 1 à 2, dans lequel ladite membrane a des pores ayant un diamètre égal à 1 µm.

4. Système selon chacune des revendications 1 à 3, dans lequel ladite lignée de podocytes de mammifère est immortalisée conditionnellement.

5. Système selon chacune des revendications 1 à 4, dans lequel ledit support solide est une plaque multi-puits.

6. Système selon chacune des revendications 1 à 5, comprenant également une troisième population de cellules sur le fond du premier support solide sélectionnée à partir du groupe constitué de cellules mésangiales, de fibroblastes, d'astrocytes et de cellules parenchymateuses.

7. Procédé pour la coculture in vitro de podocytes et de cellules endothéliales comprenant les étapes suivantes :
a) la culture d'une lignée de cellules de mammifère endothéliales microvasculaires sur le côté inférieur d'une membrane semi-perméable de PET enduite des deux côtés de collagène de type IV avec des pores ayant un diamètre dans la plage de 0,3 µm à 1,2 µm maintenue en suspension à l'intérieur d'un support solide contenant un premier milieu de culture en présence de VEGF à une concentration de 3 à 50 ng/ml, de préférence 5 ng/ml, pendant une semaine avant la préparation de podocytes en plaques ;
b) la culture d'une lignée de podocytes de mammifère en présence d'interféron gamma à 33 °C, pendant une semaine ;
c) le transfert de la lignée de podocytes de mammifère obtenue à partir de l'étape b) sur le côté supérieur de la membrane de l'étape a) et la culture dans un deuxième milieu en l'absence d'interféron gamma et à une température de 37 °C ;
d) la coculture des deux lignées de cellules dans les deux environnements séparés par la membrane.

8. Procédé selon la revendication 7, dans lequel ledit support solide est une plaque multi-puits.

9. Procédé selon chacune des revendications 7 à 8, dans lequel ladite membrane est constituée de PET avec des pores ayant un diamètre de 1 µm.

10. Procédé selon chacune des revendications 7 à 9, dans lequel ladite lignée de podocytes est immortalisée conditionnellement.

11. Procédé selon la revendication 10, dans lequel quand une ligne de podocytes immortalisée conditionnellement inductible par interféron gamma, développée en présence d'interféron gamma à 33 °C, est utilisée, la culture dans un deuxième milieu de l'étape c) s'effectue en l'absence d'interféron gamma et à 37°C.

12. Procédé selon chacune des revendications 7 à 11, dans lequel, au point b), une culture primaire de podocytes de mammifère est utilisée.

13. Procédé selon chacune des revendications 7 à 12, dans lequel ladite lignée de cellules endothéliales microvasculaires est la lignée murine ATCC CRL-2586.

14. Procédé selon chacune des revendications 7 à 13, comprenant l'introduction d'une troisième lignée de cellules sur le fond du support solide sélectionnée à partir du groupe constitué de cellules mésangiales, de fibroblastes, d'astrocytes et de cellules parenchymateuses.

15. Utilisation du système de coculture in vitro tel que défini selon les revendications 1 à 6, dans un procédé de sélection de médicaments ou substances adaptés pour modifier la fonctionnalité du glomérule rénal comprenant l'évaluation d'un ou plusieurs paramètres sélectionnés parmi la perméabilité cellulaire, la morphologie des cellules, l'immunodétection des marqueurs d'expression libérés dans les surnageants des populations cellulaires.

16. Utilisation du système de coculture in vitro selon la revendication 15, dans laquelle ladite perméabilité cellulaire est évaluée par le biais de l'utilisation d'albumine ou de dextranes, également marquées.

17. Utilisation du système de coculture in vitro selon la revendication 15, dans laquelle la morphologie des cellules est analysée par microscopie optique ou électronique.

18. Utilisation du système de coculture in vitro selon la revendication 15, dans laquelle les marqueurs d'expression libérés dans les surnageants sont détectés par le biais du test ELISA ou Western Blot.

19. Utilisation du système de coculture in vitro tel que défini selon les revendications 1 à 6, comme un modèle d'étude in vitro des dysfonctionnements ou des pathologies affectant les glomérules rénaux.

20. Utilisation du système de coculture in vitro tel que défini selon les revendications 1 à 6, dans un procédé de sélection de médicaments ou substances adaptés pour modifier la fonctionnalité du glomérule rénal comprenant l'évaluation d'un ou plusieurs paramètres sélectionnés parmi la perméabilité cellulaire, la morphologie des cellules, l'immunodétection des marqueurs d'expression libérés dans les surnageants des populations cellulaires.
